(19) Europäisches Patentamt
European Patent Office
Office européen des brevets

(11) Veröffentlichungsnummer: **0 035 219**
**B1**

(12) # EUROPÄISCHE PATENTSCHRIFT

(45) Veröffentlichungstag der Patentschrift: **02.03.83**

(51) Int. Cl.³: **C 07 D 277/72**

(21) Anmeldenummer: **81101316.8**

(22) Anmeldetag: **24.02.81**

(54) **Verfahren zur Herstellung von 2-Merkaptobenzthiazolen.**

(30) Priorität: **04.03.80 DE 3008225**

(43) Veröffentlichungstag der Anmeldung: **09.09.81 Patentblatt 81/36**

(45) Bekanntmachung des Hinweises auf die Patenterteilung: **02.03.83 Patentblatt 83/9**

(84) Benannte Vertragsstaaten: **BE CH DE FR GB IT LI NL**

(56) Entgegenhaltungen:
**DE-A-2 709 990**
**DE-A-2 816 407**
**DE-B-1 131 678**

(73) Patentinhaber: **HOECHST AKTIENGESELLSCHAFT, Postfach 80 03 20, D-6230 Frankfurt/Main 80 (DE)**

(72) Erfinder: **Handte, Reinhard, Dr., Breckenheimer Strasse 45, D-6238 Hofheim am Taunus (DE)**
Erfinder: **Willms, Lothar, Dr., Grüner Weg 4, D-5463 Unkel (DE)**
Erfinder: **Blume, Ernst, Dr., Im Hainpfad 10, D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 035 219 B1

BUNDESDRUCKEREI BERLIN

Verfahren zur Herstellung von 2-Merkaptobenzthiazolen

In der Literatur werden verschiedene Verfahren zur Herstellung von 2-Merkaptobenzthiazolen beschrieben, wobei insbesondere die Herstellung von unsubstituiertem 2-Merkaptobenzthiazol Gegenstand einer Vielzahl von Patentanmeldungen und Veröffentlichungen ist.

Die wichtigsten Herstellverfahren beruhen auf der Umsetzung von 2-Chlornitrobenzol mit Schwefelwasserstoff und Schwefelkohlenstoff bzw. auf der Umsetzung von Anilin mit Schwefel und Schwefelkohlenstoff (z. B. Ullmanns Enzyklopädie der technischen Chemie, 3. Auflage, Bd. 12, S. 304; NL-OS 7 800 037, JP 53 009-766, IT-PS 2 454 277, NL-OS 7 105 735, JP 7 121 019, JP 7 121 018, US-PS 674 091, US-PS 674 092, US-PS 674 093, DE-OS 2 709 989, US-PS 1 662 015, US-PS 1 785 656, US-PS 1 669 630, US-PS 1 960 205). In der DE-OS 2 816 503 wird zur Herstellung von 2-Merkaptobenzthiazol die Umsetzung von Nitrobenzol in Gegenwart von Schwefelwasserstoff und Schwefelkohlenstoff vorgeschlagen. Gemäß GB-PS 1 379 127, 1 386 446 und 1 404 954 lassen sich N-Methylanilin oder Anilin mit Dimethylformamid sowie Anilin mit einem aliphatischen Amin jeweils in Gegenwart von Schwefel zu 2-Merkaptobenzthiazol umsetzen. Nach den zuletzt genannten Verfahren wird der zur Cyclisierung notwendige Schwefelkohlenstoff offenbar intermediär gebildet.

Die genannten Vorveröffentlichungen beziehen sich fast ohne Ausnahme auf die Herstellung von unsubstituiertem 2-Merkaptobenzthiazol. Die Herstellung einer Reihe von substituierten 2-Merkaptobenzthiazolen wird z. B. in J. Am. Chem. Soc. 49, 1748—58 und 1779—85 (1927), sowie 56, 2734—36 (1934), beschrieben. Gemäß den älteren Veröffentlichungen werden u. a. substituierte 2-Chlornitrobenzole mittels NaSH, $H_2S$ und $CS_2$ in entsprechende 2-Mercaptobenzthiazole überführt; gemäß der letztgenannten Literaturstelle erfolgt diese Umsetzung mittels Natriumsulfid, Schwefel und Schwefelkohlenstoff.

Vermutlich besteht der Primärschritt dieser Reaktion im Austausch des durch die Nachbarstellung der $NO_2$-Gruppe aktivierten Chloratoms gegen Schwefel unter Ausbildung einer —S—S-Brücke zwischen zwei Molekülen Nitrobenzol. In einem weiteren Teilschritt wird anschließend die Nitrogruppe und die Disulfidbrücke mittels Natriumpolysulfid (aus $Na_2S$+S) reduziert, und schließlich werden aus den entstandenen *a*-Amino-thiophenolen und $CS_2$ die 2-Mercaptobenzthiazole gebildet.

Einer der Nachteile dieser Arbeitsweise liegt in den ungünstigen Mengenverhältnissen, da Natriumsulfid, Schwefel und Schwefelkohlenstoff in hohen Überschüssen angewendet werden und dementsprechend große Mengen an nicht verbrauchten Ausgangsstoffen übrigbleiben. Vor allem aber ist das Verfahren ungeeignet zur Synthese der 6-Halogenmercaptobenzthiazole, da bei der Umsetzung mit 2,4-Dichlornitrobenzol nur undefinierbare Produkte entstehen.

Zur Herstellung von 6-Chlor- bzw. 6-Brom-2-mercaptobenzthiazol ist aus der Literatur bisher nur ein Verfahren bekannt. Teppema und Sebrell (J. Am. Chem. Soc. 49, 1783 [1927]) diazotierten 6-Amino-2-mercaptobenzthiazol (das seinerseits durch Nitrierung von 2-Mercaptobenzthiazol und anschließende Reduzierung erhalten worden war) und setzen das Diazoniumsalz mit Kupfer(I)chlorid um. Unter gleichen Bedingungen erhält man nach Chem. Zvest. 27, 698—702 (1973), bei Anwendung von Kupfer(I)bromid das entsprechende Bromderivat. Die Herstellung der Verbindungen auf diesem Weg ist jedoch umständlich und technisch unbefriedigend, da die Ausbeuten und die erzielten Reinheiten nicht genügen. Versuche, durch Kernchlorierung von 2-Merkaptobenzthiazol 6-Chlor-2-merkaptobenzthiazol herzustellen, waren nicht erfolgreich (J. Am. Chem. Soc. 49, 1783 [1927]; siehe auch DP 1 168 911).

Gegenstand der vorliegenden Erfindung ist nunmehr ein neuartiges Verfahren zur Herstellung von 2-Mercaptobenzthiazolen, das dadurch gekennzeichnet ist, daß man 2-Halogenaniline mit Alkali- oder Erdalkalixanthaten oder mit Schwefelkohlenstoff — letzteres in Gegenwart von Basen — umsetzt.

Nach dem erfindungsgemäßen Verfahren lassen sich prinzipiell alle bekannten ein- oder mehrfach substituierten 2-Mercaptobenzthiazole herstellen, sofern die Reste unter den angewendeten Reaktionsbedingungen gegenüber dem Reaktionsmedium inert sind und sich sterisch nicht behindern. So kann in der Formel I

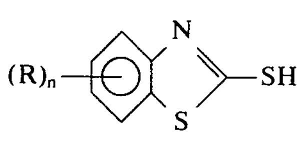

(I)

R z. B. ($C_1$—$C_4$)Alkyl, ($C_1$—$C_4$)Alkoxy, ($C_1$—$C_4$)Alkylthio, Halogen (F, Cl, Br, J), Halogen($C_1$—$C_4$)alkyl ($CF_3$), Halogen($C_1$—$C_4$)alkoxy, Phenoxy, Phenylthio, Benzyl, Benzyloxy (wobei die Phenylreste durch Halogen oder niederes Alkyl substituiert sein können), $NO_2$, OH, CN, Di($C_1$—$C_4$)alkylamino, Niederalkoxy-, Phenoxy-, Halogen- oder Alkylphenoxy-, Alkylamino-, Dialkylamino- oder Heteroaminosulfonyl und n 0 oder eine Zahl von 1—4 bedeuten. Bevorzugt wird das Verfahren zur Herstellung der auf direktem Wege bisher nicht zugänglichen 6-Chlor- und 6-Brom-2-mercaptobenzthiazole angewendet.

Beispielsweise lassen sich folgende Verbindungen nach dem erfindungsgemäßen Verfahren herstellen (vgl. auch die Verfahrensbeispiele):

4,6-Dichlor-2-mercaptobenzthiazol
7-Chlor-2-mercaptobenzthiazol
6-Ethyl-2-mercaptobenzthiazol
6-Trifluormethyl-2-mercaptobenzthiazol
6-tert.Butyl-2-mercaptobenzthiazol
6-Methoxy-2-mercaptobenzthiazol
6-Ethoxy-2-mercaptobenzthiazol
5-Ethoxy-2-mercaptobenzthiazol
5-Fluor-2-mercaptobenzthiazol
5-Brom-2-mercaptobenzthiazol
4-Chlor-2-mercaptobenzthiazol
6-Nitro-2-mercaptobenzthiazol
6-Cyano-2-mercaptobenzthiazol
6-Phenoxy-2-mercaptobenzthiazol
5-(4-Dimethylaminosulfonyl)-2-mercaptobenzthiazol
5-(4-Methoxysulfonyl)-2-mercaptobenzthiazol
5-(4-Chlorphenoxysulfonyl)-2-mercaptobenzthiazol

Das Verfahren verläuft nach folgendem Schema:

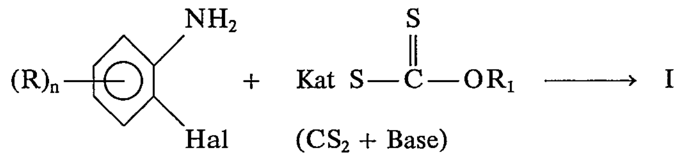


In obiger Gleichung hat $(R)_n$ die angegebene Bedeutung, Hal steht für Halogen, vorzugsweise für Fluor, Chlor oder Brom, und Kat für ein Alkali- bzw. Erdalkalikation; $R_1$ bedeutet Alkyl oder Cycloalkyl, ist aber nicht auf diese beschränkt. Bevorzugte Xanthate sind Kalium- und Natrium$(C_1-C_4)$alkylxanthat.

Daß das erfindungsgemäße Verfahren in fast quantitativer Ausbeute die gewünschten 2-Mercaptobenzthiazole liefert, war in Kenntnis des Standes der Technik nicht zu erwarten. Üblicherweise gelingt die nukleophile Substitution von Halogenatomen an Aromaten nur, wenn diese durch weitere elektrophile Gruppen (z. B. $NO_2$) ausreichend aktiviert sind. Das erfindungsgemäße Verfahren führt dagegen sogar dann zum Ziel, wenn statt aktivierender Substituenten weitere desaktivierende Reste (z. B. $CH_3$ oder $OCH_3$) vorhanden sind.

Zu der erfindungsgemäßen Umsetzung eignen sich sowohl technische Xanthate, wie sie in großem Umfang als Flotationshilfsmittel im Handel erhältlich sind, als auch frisch hergestellte Xanthate, die vor oder während der Reaktion in situ aus einem Alkohol, einer starken Base und Schwefelkohlenstoff erzeugt werden können. Bevorzugt verwendet man Natrium- und Kaliumxanthate der niederen Alkohole, doch kommen auch Erdalkalixanthate wie Kalziumxanthat in Betracht. Zur Herstellung der ersteren verwendet man bevorzugt Natron- oder Kalilauge bzw. Methanol, Ethanol, Propanol, Isopropanol, Butanol oder Isobutanol. Anstatt Base + Alkohol getrennt anzuwenden, kann man selbstverständlich auch von entsprechenden Alkalialkoholaten ausgehen. Die Umsetzung mit Schwefelkohlenstoff kann in An- oder Abwesenheit eines Alkohols erfolgen, jedoch ist hier die Anwesenheit einer Base erforderlich. Als solche eignen sich ebenfalls NaOH und KOH, ferner Alkali- und Erdalkalicarbonate und -hydrogencarbonate wie $K_2CO_3$, $Na_2CO_3$, $NaHCO_3$ oder $CaCO_3$. Das Molverhältnis der Reaktionskomponenten ist nicht kritisch und kann bei geeigneter Versuchsanstellung in weiten Grenzen variiert werden. So lassen sich die 2-Halogenaniline im Überschuß wie auch im Unterschuß einsetzen. Wird das Anilin im Überschuß eingesetzt, so kann es sich bei der Aufarbeitung durch Extraktion mit geeigneten Lösungsmittel (z. B. Toluol) zurückgewonnen werden. Aus ökonomischen Gründen werden jedoch Molverhältnisse (Xanthat bzw. Schwefelkohlenstoff zu 2-Halogenanilin) von 0,8 : 1 bis 4 : 1, insbesondere 1 : 1 bis 3 : 1, bevorzugt. Bei Umsetzungen im äquimolaren Bereich ist zur Erzielung eines hohen Umsatzes an 2-Halogenanilin ein Zusatz einer Hilfsbase zur Neutralisation des entstehenden 2-Mercaptobenzthiazols von Vorteil. Geeignete Basen sind beispielsweise Alkalicarbonate, die dann in mindestens äquivalenten Mengen eingesetzt werden. Da ein Überschuß an Alkalicarbonat sich positiv auf die Ausbeute auswirkt, liegt der bevorzugte Alkalicarbonatzusatz in der Größenordnung von 0,5 Mol bis 4 Mol je Mol eingesetztes Halogenanilin. Die Reaktionstemperatur kann innerhalb weiter Grenzen variiert werden. Aus praktischen Gründen wählt man eine Temperatur im Bereich von 80°C bis 250°C, wobei der Bereich von 100°C bis 170°C bevorzugt ist. Zweckmäßigerweise wird bei dem Verfahren in Anwesenheit eines Lösungsmittels gearbeitet. Geeignet sind vor allem hochsiedende polare aprotische Lösungsmittel wie Dimethylformamid, Dimethylacetamid, N-Methylpyrrolidon, Dimethylsulfoxyd oder Sulfolan. Die Reaktionszeit richtet sich nach den Bedingungen, unter denen das erfindungsgemäße Verfahren durchgeführt wird. In der Regel liegt sie zwischen zwei und vierundzwanzig Stunden.

Üblicherweise wird die Umsetzung bei Normaldruck durchgeführt, es ist jedoch auch möglich, insbesondere bei der Verwendung von freiem Schwefelkohlenstoff, im geschlossenen Reaktionsgefäß zu

arbeiten. Der sich aufbauende Druck hängt dann von der gewählten Reaktionstemperatur ab.

Die nach dem erfindungsgemäßen Verfahren hergestellten 2-Mercaptobenzthiazole werden gewünschtenfalls nach erfolgter Cyclisierung durch Zusatz von Säuren aus den zunächst anfallenden Salzen freigesetzt. Für einige Folgereaktionen nach dem erfindungsgemäßen Verfahren ist die Anwendung freier Mercaptane nicht erforderlich; so lassen sich z. B. Chlorierungen, Alkylierungen oder auch Oxydationen direkt mit den Salzen durchführen. Die Aufarbeitung kann durch Abdestillieren des Lösungsmittels und/oder durch Zugabe von Wasser vor dem Ansäuern erfolgen. Die freien Mercaptane werden anschließend durch Filtration isoliert.

Die 2-Mercaptobenzthiazole eignen sich als reaktive Thiole für vielfältige Zwecke. Sie lassen sich z. B. als Vulkanisationshilfsmittel oder auch als Ausgangsstoffe für die Herstellung von Farbstoffen, Pharmazeutika und Pflanzenschutzmitteln verwenden (vgl. zum Beispiel Jap. Kokai 7 210 124, JP-AS 7 123 015; GB-PS 1 153 648, DE-OS 2 640 730 und DE-OS 2 531 427).

Die nachfolgenden Beispiele erläutern das erfindungsgemäße Verfahren:

Beispiel 1

a) Zu einer auf 140—150°C erhitzten Lösung von 63,8 g 2-Chloranilin in 150 ml Dimethylformamid läßt man unter Stickstoffatmosphäre innerhalb einer Stunde eine Lösung von 216,2 g Natriummethylxanthat in 250 ml Dimethylformamid zutropfen. Die Innentemperatur wird dabei durch teilweise Abnahme von Destillat bei 150°C gehalten. Anschließend wird das Reaktionsgemisch ca. 12 Stunden unter Rückfluß nachgerührt, auf ca. 80°C abgekühlt und in 1,2 l Eiswasser eingerührt. Die erhaltene trübe Lösung wird angesäuert und das ausgefallene 2-Mercaptobenzthiazol abgesaugt. Nach Waschen und Trocknen erhält man 79 g (94,6% d. Th.) 2-Mercaptobenzthiazol vom Fp. 179—180°C.

b) In analoger Weise erhält man nach $2^1/_2$stündiger Reaktionszeit 6-Chlor-2-mercaptobenzthiazol (Fp. 260—262°C) in 95,6%iger Ausbeute.

Die Verfahrensbedingungen können gemäß nachstehender Tabelle innerhalb weiter Grenzen variiert werden: Erläuterungen zur Tabelle:

Tabelle 1

6-Chlor-2-mercaptobenzthiazol, hergestellt durch Umsetzung von 2,4-Dichloranilin mit Xanthaten

| Molverhältnis Anilin/Xanthat | Xanthat ① | LM ② | Basenzusatz | Reaktionszeit (gesamt) h | Ausbeute % | Fp (° C) |
|---|---|---|---|---|---|---|
| 1 : 3 | NaEx | DMSO | — | 4 | 93,7 | ~256 |
| 1 : 3 | NaEx | DMAA | — | 4 | 94,3 | 254—256 |
| 1 : 3 | NaEx | NMP | — | 4 | 91 | 257 |
| 1 : 3 | KEx | DMF | — | 3,5 | 95,2 | ~260 |
| 1 : 2 | NaIBX | DMF | 1 Äquivalent $K_2CO_3$ | 20 | 96,3 | ~261 |
| 1 : 1,1 | NaIBX | DMF | 2 Äquivalente $CaCO_3$ | 24 | 94,8 | ~258—260 |
| 1 : 1,2 | KIBX | DMF | 2,5 Äquivalente $Na_2CO_3$ | 22 | 87,5 | ~257 |
| 1 : 3<br>1 : 0,8 | NaIPX | DMF | — | 4 | 92,8 | ~258 |

① NaEx = Natriumethylxanthat
KEx = Kaliumethylxanthat
NaIBX = Natriumisobutylxanthat
NaIPX = Natriumisopropylxanthat

LM = Lösungsmittel:
② DMSO = Dimethylsulfoxyd
DMAA = Dimethylacetamid
NMP = N-Methylpyrrolidon
DMF = Dimethylformamid

Beispiel 2

a) Zu einer gerührten Suspension von 135 g Natriummethylat in 1,3 l Dimethylformamid werden unter Stickstoffatmosphäre 205 g Schwefelkohlenstoff tropfenweise zugegeben. Die Temperatur der Reaktionsmischung steigt dabei auf ca. 70°C an. Bei einer Innentemperatur von 70—80°C läßt man im Verlauf von ca. 30—60 Minuten eine Lösung von 288 g 2,4-Dibromanilin in 300 ml Dimethylformamid zutropfen. Danach wird unter guter Rückflußkühlung die Innentemperatur auf 100°C gesteigert und ca. 14 Stunden beibehalten, anschließend wird abgekühlt, das Reaktionsgemisch in 6 l Wasser eingerührt und angesäuert. Das ausfallende 6-Brom-2-mercaptobenzthiazol wird abgesaugt und mit Wasser gewaschen. Nach dem Trocknen im Vakuum erhält man 272 g (96,3% d. Th., bezogen auf eingesetztes 2,4-Dibromanilin) an 6-Brom-2-mercaptobenzthiazol, dessen Reinheit für weitere Umsetzungen ausreicht. Eine gereinigte Probe besaß einen Fp. von 278°C.

b) In analoger Weise erhält man bei Verwendung von 2,5-Dichloranilin nach 17stündiger Reaktion das 5-Chlor-2-mercaptobenzthiazol (Fp. 191—193°C) in 93,5%iger Ausbeute.

c) In analoger Weise erhält man bei Verwendung von 2,4,5-Trichloranilin das 5,6-Dichlor-2-mercaptobenzthiazol (Fp. 234—236°C) in 88%iger Ausbeute.

d) In analoger Weise erhält man bei Verwendung von 2,4-Dichloranilin nach 16stündiger Reaktion das 6-Chlor-2-mercaptobenzthiazol (Fp. 257—295°C) in 91%iger Ausbeute.

Beispiel 3

a) Eine gerührte Suspension von 162 g 2,4-Dichloranilin und 414 g Kaliumcarbonat in 1000 ml Dimethylformamid wird unter Stickstoffatmosphäre innerhalb von 40 Minuten bei ca. 25—30°C tropfenweise mit 91 g Schwefelkohlenstoff versetzt. Dann wird innerhalb von 2 Stunden auf eine Innentemperatur von 150°C erwärmt und die Temperatur 16 Stunden beibehalten. Der Ansatz wird abgekühlt, abfiltriert und das Filtrat weitgehend von Dimethylformamid befreit. Der verbleibende Rückstand wird mit 600 ml Wasser versetzt und angesäuert, das ausfallende Produkt wird abfiltriert und getrocknet. Man erhält 187,6 g (93,2% d. Th.) 6-Chlor-2-mercaptobenzthiazol, Fp. 258°C.

b) In analoger Weise erhält man bei Verwendung von 2-Chlor-4-methylanilin das 6-Methyl-2-mercaptobenzthiazol (Fp. 179—181°C) in 90,7%iger Ausbeute.

Beispiel 4

Zu einer Mischung von 141,5 g 2-Chlor-6-methylanilin und 276 g Kaliumcarbonat in 1 Liter Dimethylformamid wird unter Stickstoffatmosphäre bei 150°C innerhalb einer Stunde eine Lösung von 250 g Kaliumisobutylxanthat (ca. 90%ig) in 400 ml Dimethylformamid tropfenweise zugegeben. Unter intensiver Kühlung wird das Reaktionsgemisch noch ca. 26 Stunden gerührt. Der Ansatz wird abgekühlt, vom Salz befreit und anschließend zur Trockene eingeengt. Der Rückstand wird in 400 ml Wasser aufgenommen und angesäuert. Das ausgefallene 4-Methyl-2-mercaptobenzthiazol wird abgesaugt, mit Wasser gewaschen und getrocknet. Man erhält 172 g (95% d. Th.) 4-Methyl-2-mercaptobenzthiazol, Fp. 188—190°C.

Beispiel 5

326 g (2 Mol) 2,4-Dichloranilin werden mit 331 g (2,4 Mol) Kaliumcarbonat in 1,5 Liter Dimethylformamid unter Stickstoffatmosphäre auf 150°C erwärmt, anschließend wird im Verlauf von 1,5 Stunden eine Lösung von 384 g Natriumethylxanthat (ca. 90%) in 600 ml DMF tropfenweise zugegeben. Das abdestillierende Gemisch aus DMF, Ethanol und $CS_2$ wird über eine Dosierpumpe kontinuierlich in das Reaktionsgemisch zurückgeführt. Nach ca. 20 Stunden ist kein $CS_2$ mehr im Destillat nachweisbar. Das Reaktionsgemisch wird auf ca. 60°C abgekühlt, vom Salz befreit und im Vakuum eingeengt. Der erhaltene Rückstand wird in 4 Liter Wasser aufgenommen und auf pH 3—4 angesäuert. Das ausgefallene 6-Chlor-2-mercaptobenzthiazol wird abgesaugt, mit Wasser gewaschen und getrocknet. Ausbeute 391 g (97%), Fp. 262—265°C.

Bei Verwendung von 182 g $CS_2$ in Ethanol (statt Na-ethylxanthat) erhält man in sonst gleicher Weise 381 g (94% d. Th.) 6-Chlor-2-mercaptobenzthiazol vom Fp. 260—264°C.

Einige Variationen des erfindungsgemäßen Verfahrens sind in der folgenden Tabelle 2 (Beispiel 6) zusammengefaßt:

Tabelle 2

| Beisp. Nr. | $(R)_n$ | Molverhältnis Anilin/Xanthat | Xanthat ① | Lösungsmittel ② | Basenzusatz | Reaktions-zeit (ges.) [h] | Ausbeute [%] | Fp. (C°) |
|---|---|---|---|---|---|---|---|---|
| 6 a | H | 1:3 | NaIBX | DMSO | – | 12 | 95,2 | 179–181 |
| b | H | 1:2 | KIBX | DMAA | 1 Äquiv. $K_2CO_3$ | 20 | 93,8 | 178–180 |
| c | 5-$CH_3$ | 1:3 | NaIBX | DMF | – | 16 | 91,2 | 173 |
| d | 5,6-Di$CH_3$ | 1:3 | NaEX | DMF | – | 22 | 92,6 | |
| e | 6-$CH_3$ | 1:1,2 | NaEX | DMF | 4 Äquiv. $K_2CO_3$ | 20 | 96,2 | 179–181 |
| f | 6-F | 1:3 | NaEX | DMF | – | 16 | 92,5 | 226–228 |
| g | 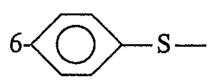 | 1:3 | NaEX | DMF | – | 18 | 87,6 | 165–167 |
| h | 6-$C_4H_9$—O— | 1:3 | NaEX | DMF | – | 20 | 84,5 | 156–157 |
| i | 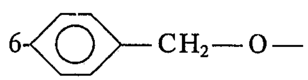 | 1:3 | NaEX | DMF | – | 20 | 78,5 | 208–212 |
| k | 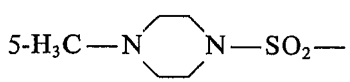 | 1:3 | NaEX | DMF | – | 14 | 96 | 250–252 |
| l | 5-$CF_3$ | 1:3 | NaIPX | DMSO | – | 12 | 92 | 208–210 |

① NaEX = Natriumethylxanthat
KIBX = Kaliumisobutylxanthat
② DMSO = Dimethylsulfoxid
DMF = Dimethylformamid
DMAA = Dimethylacetamid

**Patentansprüche**

1. Verfahren zur Herstellung von (gegebenenfalls substituiertem) 2-Mercaptobenzthiazol, dadurch gekennzeichnet, daß man ein entsprechendes 2-Halogenanilin mit einem Alkali- oder Erdalkalixanthat oder mit Schwefelkohlenstoff — letzteres in Anwesenheit einer Base — umsetzt.

2. Verfahren nach Anspruch 1, dadurch gekennzeichnet, daß man 2-Halogenanilin und Alkali- oder Erdalkalixanthat in Gegenwart von basischen Substanzen umsetzt.

3. Verfahren nach Anspruch 1, dadurch gekennzeichnet, daß man als Basen Alkalihydroxide, Alkali- oder Erdalkalicarbonate oder Alkali-hydrogen-carbonate einsetzt.

4. Verfahren nach Anspruch 1, dadurch gekennzeichnet, daß man Kaliumcarbonat als Base einsetzt.

5. Verfahren nach Anspruch 1—4, dadurch gekennzeichnet, daß man die Umsetzung in einem polaren aprotischen Lösungsmittel durchführt.

6. Verfahren nach Anspruch 1—5, dadurch gekennzeichnet, daß man die Umsetzung bei Temperaturen von 100—170°C durchführt.

7. Verfahren nach Anspruch 1—6, dadurch gekennzeichnet, daß man eingesetzten oder während der Reaktion thermisch gebildeten Schwefelkohlenstoff zur Vervollständigung der Reaktion in die Flüssigphase zurückführt.

**Claims**

1. A process for the preparation of (optionally substituted) 2-mercaptobenzothiazole, which comprises reacting a corresponding 2-halogenoaniline with an alkali metal xanthate or alkaline earth metal xanthate or with carbon disulfide, in the latter case in the presence of a base.

2. A process as claimed in claim 1, wherein the 2-halogenoaniline and alkalimetal xanthate or alkaline earth metal xanthate are reacted in the presence of basic substances.

3. A process as claimed in claim 1, wherein alkali metal hydroxides, alkali metal carbonates, alkaline earth metal carbonates or alkali metal bicarbonates are use as the bases.

4. A process as claimed in claim 1, wherein potassium carbonate is used as the base.

5. A process as claimed in any of claims 1 to 4, wherein the reaction is carried out in a polar aprotic solvent.

6. A process as claimed in any of claims 1 to 5, wherein the reaction is carried out at temperatures from 100 to 170°C.

7. A process as claimed in any of claims 1 to 6, wherein carbon disulfide which is employed or is formed under the influence of heat during the reaction is recycled into the liquid phase in order to bring the reaction to completion.

**Revendications**

1. Procédé pour préparer le mercapto-2 benzothiazole ou un dérivé de substitution de celui-ci, procédé caractérisé en ce qu'on fait réagir une halogéno-2 aniline correspondante avec un xanthate de métal alcalin ou de métal alcalinoterreux ou avec du sulfure de carbone, en opérant, dans ce dernier cas, en présence d'une base.

2. Procédé selon la revendication 1, caractérisé en ce qu'on fait réagir une halogéno-2 aniline et un xanthate de métal alcalin ou de métal alcalinoterreux, en présence de substances basiques.

3. Procédé selon la revendication 1, caractérisé en ce qu'on utilise, comme bases, des hydroxydes de métaux alcalins, des carbonates de métaux alcalins ou de métaux alcalinoterreux ou des hydrogénocarbonates de métaux alcalins.

4. Procédé selon la revendication 1, caractérisé en ce qu'on utilise, comme base, le carbonate de potassium.

5. Procédé selon l'une quelconque des revendications 1 à 4, caractérisé en ce qu'on effectue la réaction dans un solvant aprotique polaire.

6. Procédé selon l'une quelconque des revendications 1 à 5, caractérisé en ce qu'on effectue la réaction à des températures de 100 à 170°C.

7. Procédé selon l'une quelconque des revendications 1 à 6, caractérisé en ce que le sulfure de carbone mis en jeu ou formé par voie thermique au cours de la réaction est renvoyé dans la phase liquide pour compléter la réaction.